(19) Europäisches Patentamt
European Patent Office
Office européen des brevets

(11) Veröffentlichungsnummer: **0 053 321**
**B1**

(12) # EUROPÄISCHE PATENTSCHRIFT

(45) Veröffentlichungstag der Patentschrift: **29.05.85**

(21) Anmeldenummer: **81109729.4**

(22) Anmeldetag: **17.11.81**

(51) Int. Cl.⁴: **C 07 C 79/46,** C 07 C 121/75, C 07 C 79/36, C 07 C 79/22, C 07 C 79/35, A 01 N 37/48, A 01 N 37/34

(54) **Substituierte Phenoxyzimtsäurederivate, Verfahren zu deren Herstellung und deren Verwendung als Herbizide.**

(30) Priorität: **28.11.80 DE 3044810**

(43) Veröffentlichungstag der Anmeldung: **09.06.82 Patentblatt 82/23**

(45) Bekanntmachung des Hinweises auf die Patenterteilung: **29.05.85 Patentblatt 85/22**

(84) Benannte Vertragsstaaten: **AT BE CH DE FR GB IT LI NL**

(56) Entgegenhaltungen:
**DE - A - 2 304 006**
**DE - A - 2 753 900**
**GB - A - 1 139 138**
**US - A - 3 910 984**

(73) Patentinhaber: **BAYER AG, Konzernverwaltung RP Patentabteilung, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert, Dr., Im Waldwinkel 110, D-5060 Bergisch-Gladbach 2 (DE)**

**EP 0 053 321 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue substituierte Phenoxyzimtsäurederivate, ein Verfahren zu deren Herstellung und deren Verwendung als Herbizide.

Es ist bekannt, daß bestimmte substituierte Diphenylether zur Unkrautbekämpfung verwendet werden können (vgl. R. Wegler, Chemie der Pflanzenschutz- und Schädlingsbekämpfungsmittel, Band 5, S. 73—80, Springer-Verlag Berlin, Heidelberg, New York 1977).

Die Wirkung der als Herbizide vorbekannten Verbindungen aus der Reihe der Diphenylether ist jedoch für viele Zwecke nicht zufriedenstellend.

Es wurden nun neue substituierte Phenoxyzimtsäurederivate der Formel

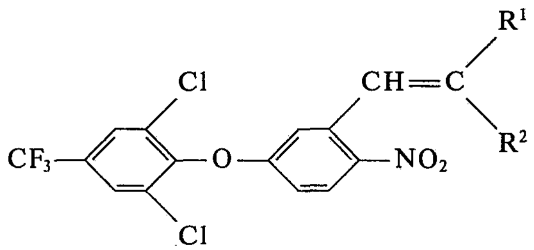

(I)

in welcher

$R^1$ für Wasserstoff, Cyano, $C_1$—$C_4$-Alkyl, Acetyl oder $C_1$—$C_4$-Alkoxycarbonyl steht und
$R^2$ für Cyano, $C_1$—$C_4$-Alkoxy-carbonyl oder COOM steht, wobei M für Wasserstoff, Natrium, Kalium, ein Magnesium- oder Calciumionenäquivalent steht,

gefunden.

Weiter wurde gefunden, daß man die neuen Phenoxyzimtsäurederivate der Formel (I) erhält, wenn man den substituierten Phenoxybenzaldehyd der formel

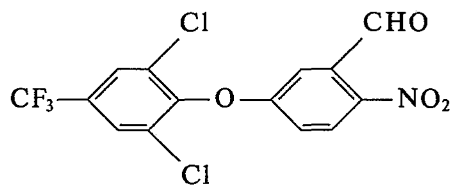

(II)

mit Methylenverbindungen der Formel

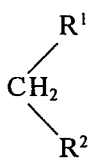

(III)

in welcher

$R^1$ und $R^2$ die oben angegebene Bedeutung haben und
$R^1$ außerdem auch für Carboxyl steht,

gegebenenfalls in Anwesenheit eines Katalysators und gegebenenfalls in Anwesenheit eines Verdünnungsmittels umsetzt und gegebenenfalls die hierbei gebildeten Verbindungen der Formel (I) nach üblichen Methoden zur Abwandlung der Reste $R^1$ und/oder $R^2$ in andere Verbindungen der Formel (I) umwandelt.

Schließlich wurde gefunden, daß die neuen Phenoxyzimtsäurederivate der Formel (I) sich durch hervorragende herbizide Wirksamkeit auszeichnen und eine gute Selektivität in verschiedenen Kulturen wie Sojabohnen, Mais und Getreide zeigen. Neben der hohen Wirkung gegen breitblättrige Unkräuter zeigen die erfindungsgemäßen Verbindungen auch eine gute Wirkung gegen Gräser, vor allem gegen Hirsearten; sie zeigen ferner bei Nachauflaufanwendung pflanzenwuchsregulierende, insbesondere wuchshemmende Eigenschaften und können beispielsweise als Baumwolldefoliants genutzt werden.

Überraschenderweise zeigen die erfindungsgemäßen Phenoxyzimtsäurederivate der Formel (I) eine wesentlich bessere herbizide Wirkung als aus dem Stand der Technik bekannte Verbindungen analoger Struktur und gleicher Wirkungsrichtung.

Verwendet man bei dem erfindungsgemäßen Verfahren 5-(2,6-Dichlor-4-trifluormethyl-phenoxy)-2-nitro-benzaldehyd und Malonsäuredimethylester als Ausgangsstoffe, so kann der Verlauf der Reaktion durch das folgende Formelschema wiedergegeben werden:

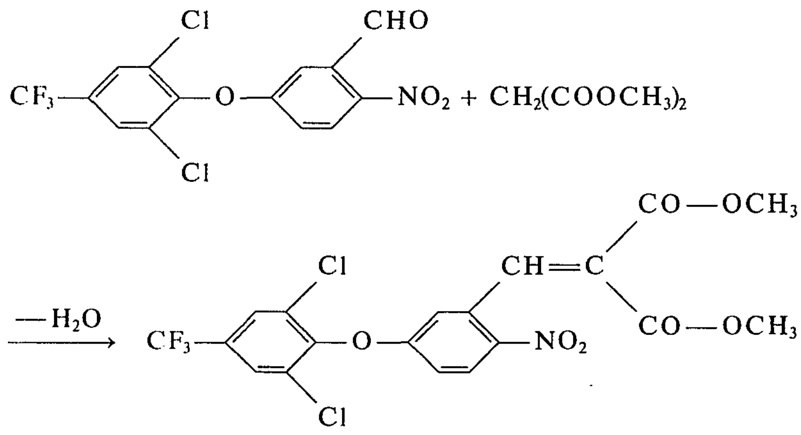


Den bei dem erfindungsgemäßen Verfahren als Ausgangsstoff zu verwendenden Phenoxybenzaldehyd der Formel (II) erhält man, wenn man

(a) das Phenoxybenzaldehyd-acylal der Formel

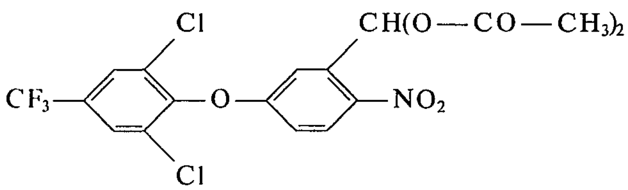
(IV)

mit wäßrigen Alkalilaugen, wie z. B. Natronlauge, gegebenenfalls in Gegenwart eines weiteren Verdünnungsmittels, wie z. B. Methanol, bei Temperaturen zwischen 10 und 80°C umsetzt, danach mit Wasser verdünnt und das kristalline Produkt der Formel (II) durch Filtration isoliert, oder wenn man

(b) den Phenoxybenzylalkohol der Formel

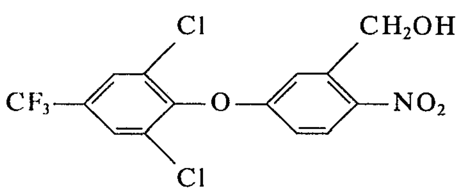
(V)

mit Oxidationsmitteln, wie z. B. Salpetersäure in Gegenwart von Verdünnungsmitteln, wie z. B. Wasser und Nitromethan, bei Temperaturen zwischen 10 und 80°C umsetzt, danach mit Wasser verdünnt und das kristalline Produkt der Formel (II) durch Filtration isoliert.

Das Acylal der Formel (IV) ist noch nicht in der Literatur beschrieben. Man erhält diese Verbindung, wenn man den Phenoxybenzaldehyd der Formel

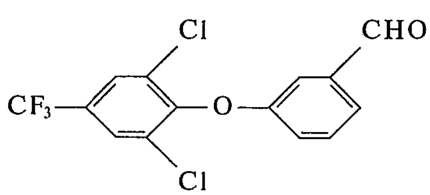
(VI)

mit Acetanhydrid, gegebenenfalls in Gegenwart eines Verdünnungsmittels, wie z. B. Methylenchlorid, bei Temperaturen zwischen 10 und 80°C umsetzt, dann Salpetersäure bei Temperaturen zwischen −10 und +20°C dazu gibt, nach Ende der Umsetzung mit Wasser verdünnt, gegebenenfalls das nichtwäßrige Verdünnungsmittel abtrennt oder abdestilliert und das kristallin anfallende Produkt der Formel (IV) durch Filtration isoliert.

Der Phenoxybenzaldehyd der Formel (VI) ist noch nicht in der Literatur beschrieben. Man erhält diese Verbindung, wenn man 3-Hydroxy-benzaldehyd mit 3,4,5-Trichlorbenzotrifluorid in Gegenwart eines

Säurebindemittels, wie z. B. Kaliummethylat und in Gegenwart von Verdünnungsmitteln, wie z. B. Dimethyl sulfoxid und Toluol, bei Temperaturen zwischen 50 und 120°C umsetzt. Die Aufarbeitung kann nach üblichen Methoden durchgeführt werden, beispielsweise durch Abdestillieren der flüchtigen Komponenten, Digerieren des Rückstandes mit Toluol, Filtration, Waschen und Einengen des Filtrats. Der hierbei erhaltene Rückstand enthält im wesentlichen das Produkt der Formel (VI), welches über sein Bisulfit-Addukt gereinigt werden kann.

Der Phenoxybenzylalkohol der Formel (V) ist noch nicht in der Literatur beschrieben. Man erhält diese Verbindung, wenn man das Phenoxybenzoesäurechlorid der Formel

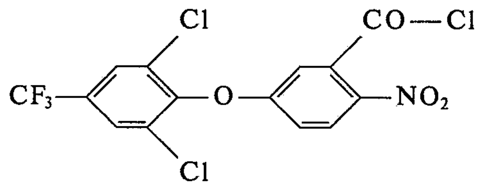

(VII)

mit Hydridkomplexen, wie z. B. Natriumboranat in Gegenwart von Verdünnungsmitteln, wie z. B. Diglycoldimethylether (Diglyme) bei Temperaturen zwischen −20 und +20°C umsetzt.

Die Aufarbeitung kann wie üblich durchgeführt werden, beispielsweise durch Verdünnen mit Wasser, Ansäuern, z. B. mit Essigsäure und Isolierung des kristallin anfallenden Produktes der Formel (V) durch Filtration.

Das Phenoxybenzoesäurechlorid der Formel (VII) ist in der Literatur beschrieben (vgl. DE-A 29 50 401).

Als Beispiele für die bei dem erfindungsgemäßen Verfahren weiterhin als Ausgangsstoffe benötigten Methylenverbindungen der Formel (III) seien genannt:

Malonsäure, deren Natrium-, Kalium-, Calcium- und Magnesiumsalz sowie deren Methylester, Ethylester, n- und iso-Propylester, n-, iso-, sek- und tert.-Butylester, Malonsäuredinitril, Cyanessigsäure, deren Natrium-, Kalium-, Calcium- und Magnesiumsalz sowie deren Methylester, Ethylester, n- und iso-Propylester, n-, iso-, sek- und tert.-Butylester, ferner Acetessigsäuremethylester, -ethylester, -n- und -iso-propylester, -n-, -iso-, sek- und -tert.-butylester.

Die Verbindungen der Formel (III) sind bekannt.

Aus Verbindungen der Formel (I), in welcher $R^2$ für Carboxyl oder Alkoxycarbonyl steht, können andere Verbindungen der Formel (I) nach üblichen Methoden hergestellt werden.

Als Möglichkeiten zur Umwandlung der nach dem erfindungsgemäßen Verfahren gebildeten Verbindungen der Formel (I) seien genannt:

(1) die Decarboxylierung, welche bei Verwendung von Malonsäure oder von Cyanessigsäure als Ausgangsstoffen im allgemeinen in situ abläuft, ohne daß die entsprechenden Primärprodukte isoliert werden;
(2) die Salzbildung, beispielsweise durch Umsetzung der entsprechenden Säuren mit basischen Alkali- oder Erdalkaliverbindungen;
(3) die Veresterung, beispielsweise durch Umsetzung der entsprechenden Säuren mit Alkoholen in Gegenwart von Basen, wie z. B. Diazabicycloundecen, oder durch Umsetzung mit geeigneten Alkylhalogeniden.

Das Verfahren zur Herstellung der neuen Phenoxyzimtsäurederivate der Formel (I) wird in Gegenwart eines Katalysators durchgeführt. Als solche dienen Verbindungen, welche für das Beschleunigen von Kondensationsreaktionen zwischen Aldehyden und aktivierten Methylenverbindungen (Aldolkondensationen) geeignet sind. Vorzugsweise werden aliphatische, aromatische oder heterocyclische Amine, wie z. B. Trimethylamin, Triethylamin, Pyrrolidin, Piperidin, Dimethylanilin, Dimethylbenzylamin, Diazabicyclooctan, Diazabicyclononen, Diazabicycloundecen und Pyridin, gegebenenfalls als Salze von schwachen Säuren, wie z. B. von Essigsäure verwendet. Zu den Katalysatoren gehören auch Aminocarbonsäuren, wie z. B. β-Alanin. Einzelne dieser Katalysatoren können, wie z. B. Pyridin, auch zweckmäßig als Verdünnungsmittel verwendet werden.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methyl-ethyl-, Methylisopropyl- und Methyl-isobutylketon, Ester, wie Essigsäure-methylester und -ethylester, Nitrile, wie z. B. Acetonitril und Propionitril, wie z. B. Dimethylformamid, Dimethylacetamid und N-Methyl-pyrrolidon, sowie Dimethylsulfoxid, Tetramethylensulfon und Hexamethyl-phosphorsäuretriamid.

Die Reaktionstemperatur kann innerhalb eines größeren Bereichs variiert werden. Im allgemeinen

arbeitet man zwischen 0 und 200°C, vorzugsweise zwischen 20 und 150°C.

Das erfindungsgemäße Verfahren wird im allgemeinen bei Normaldruck durchgeführt.

Pro Mol Phenoxybenzaldehyd der Formel (II) werden bei der Durchführung des erfindungsgemäßen Verfahrens zwischen 0,9 und 1,5 Mol, vorzugsweise zwischen 1,0 und 1,2 Mol Methylenverbindung der Formel (III) eingesetzt.

Zur Durchführung des erfindungsgemäßen Verfahrens werden im allgemeinen die Reaktionskomponenten der Formeln (II) und (III) sowie der Katalysator und das Verdünnungsmittel bei Raumtemperatur zusammengegeben, und das Gemisch wird dann bei erhöhter Temperatur, vorzugsweise zwischen 50 und 120°C bis zum Ende der Umsetzung gerührt. Das bei der Reaktion gebildete Wasser wird gegebenenfalls über einen Wasserabscheider entfernt.

Die Aufarbeitung kann nach üblichen Methoden durchgeführt werden. Soweit die Produkte bei Raumtemperatur kristallin sind, erhält man sie durch Verdünnen der Reaktionsmischung mit Wasser, gegebenenfalls Ansäuern und Filtrieren.

Es kann jedoch auch, wie üblich, durch Verdünnen mit Wasser, Schütteln mit einem mit Wasser praktisch nicht mischbaren organischen Lösungsmittel, wie z. B. Toluol, Abtrennen und Waschen der organischen Phase mit Wasser sowie Abdestillieren des organischen Lösungsmittels aufgearbeitet werden.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen, z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykolether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im preemergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,05 und 10 kg Wirkstoff pro ha, vorzugsweise zwischen 0,1 und 5 kg/ha.

Die erfindungsgemäßen Wirkstoffe zeigen bei Nachauflaufanwendung wachstumsregulierende Eigenschaften.

## Herstellungsbeispiele

### Beispiel 1

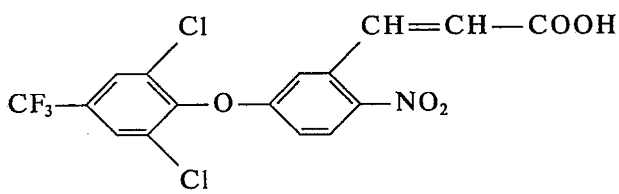

(I-1)

270 g 5-(2,6-Dichlor-4-trifluormethyl-phenoxy)-2-nitrobenzaldehyd und 6 g Piperidin werden bei 25°C zu 89,5 g Malonsäure in 570 ml Pyridin gegeben. Die Reaktionsmischung wird 5 Stunden bei 85 bis 90°C gerührt und weitere 90 Minuten unter Rückfluß erhitzt, dann auf Wasser gegossen, mit Salzsäure angesäuert und abgesaugt. Das kristalline Produkt wird aus n-Butanol umkristallisiert. Man erhält 200 g (66% der Theorie) 3-(5-(2,6-Dichlor-4-trifluormethyl-phenoxy)-2-nitro-phenyl)-propensäure vom Schmelzpunkt 192°C in Form gelber Kristalle.

Nach der im Beispiel 1 angegebenen Methode werden auch die in den folgenden Beispielen aufgeführten Stoffe der Formel (I) hergestellt:

### Beispiel 2

Unter Verwendung von Cyanessigsäure

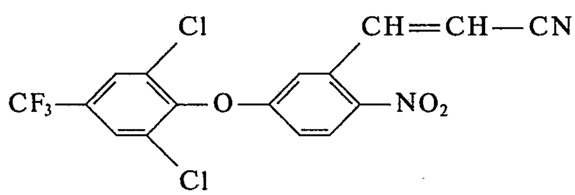

(I-2)

Schmelzpunkt: 142°C.

Beispiel 3

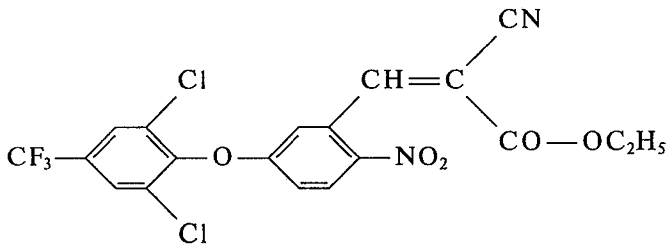
(I-3)

19 g 5-(2,6-Dichlor-4-trifluormethyl-phenoxy)-2-nitro-benzaldehyd, 5,7 g Cyanessigsäuremethylester, 0,6 g Essigsäure, 0,3 g Piperidin und 120 ml Toluol werden vermischt und 6 Stunden am Wasserabscheider zum Sieden erhitzt. Dann werden 200 ml Toluol dazugegeben, und die organische Phase wird mit Wasser gewaschen. Von der organischen Phase wird das Lösungsmittel abdestilliert und der Rückstand durch Digerieren mit Methanol zur Kristallisation gebracht. Man erhält 12,5 g (53% der Theorie) 2-Cyano-3-(5-(2,6-dichlor-4-trifluormethylphenoxy)-2-nitro-phenyl)-propen-säure-ethylester vom Schmelzpunkt 99°C in Form blaßgelber Kristalle.

Nach der im Beispiel 3 angegebenen Methode werden auch die in den folgenden Beispielen aufgeführten Stoffe der Formel (I) hergestellt:

Beispiel 4

Unter Verwendung von Malodinitril:

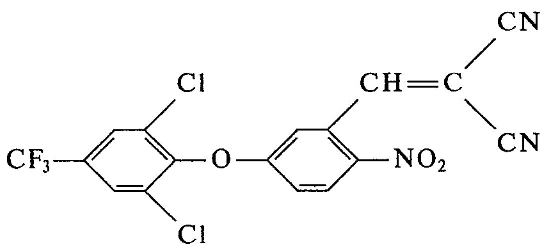
(I-4)

Schmelzpunkt: 114°C.

Beispiel 5

Unter Verwendung von Cyanessigsäuremethylester:

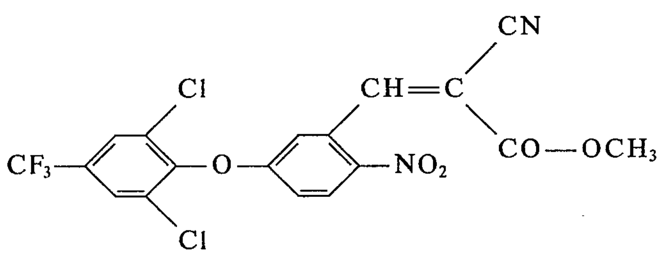
(I-5)

Schmelzpunkt: 148°C.

Beispiel 6

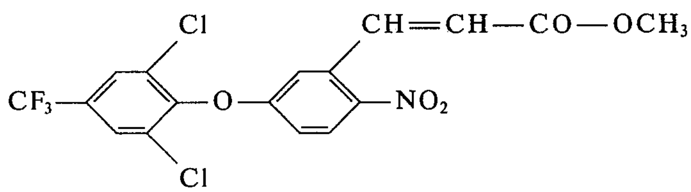
(I-6)

42,2 g (3-(5-(2,6-Dichlor-4-trifluormethyl-phenoxy)-2-nitro-phenyl)-propensäure (Verbindung I-1) werden in 120 ml Aceton vorgelegt und bei 20°C mit 18,7 g Diazabicycloundecen (DBU) versetzt.

Nach 30 Minuten werden 35 g Methyliodid tropfenweise dazugegeben.

Das Reaktionsgemisch wird 15 Stunden bei 50—60°C gerührt mit Wasser und Methylenchlorid verdünnt; die organische Phase wird mit verdünnter Natronlauge und dann mit verdünnter Salzsäure ausgeschüttelt, mit Wasser gewaschen, getrocknet und filtriert. Vom Filtrat wird das Lösungsmittel abdestilliert, der Rückstand mit Ligroin digeriert und abgesaugt.

Man erhält 20,5 g (47% der Theorie) 3-(5-(2,6-Dichlor-4-trifluormethyl-phenoxy)-2-nitro-phenyl)-propensäuremethylester vom Schmelzpunkt 90°C.

Nach der im Beispiel 6 angegebenen Methode werden auch die in den folgenden Beispielen aufgeführten Stoffe der Formel (I) hergestellt:

Beispiel 7

Unter Verwendung von 2-Jod-propan:

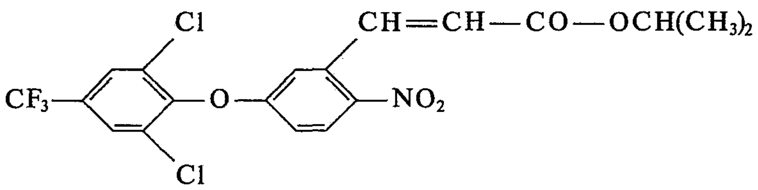

(I-7)

Schmelzpunkt: 102°C.

Beispiel 8

Unter Verwendung von Ethyljodid:

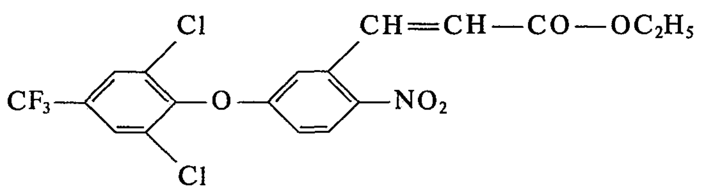

(I-8)

Schmelzpunkt: 86°C.

Herstellung von Ausgangsstoffen

Beispiel II-1

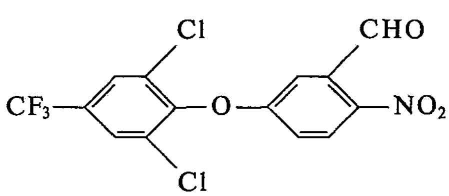

Verfahrensvariante (a)

Eine Mischung aus 12,1 g 5-(2,6-Dichlor-4-trifluormethyl-phenoxy)-2-nitro-benzaldehyd-diacetacylal, 40 ml Methanol, 30 ml Wasser und 7 ml 4%iger wäßriger Natronlauge wird 90 Minuten bei 40°C gerührt, dann in Wasser gegossen und abgesaugt. Man erhält 9,0 g (95% der Theorie) 5-(2,6-Dichlor-4-trifluormethyl-phenoxy)-2-nitro-benzaldehyd vom Schmelzpunkt 115°C.

Beispiel IV-1

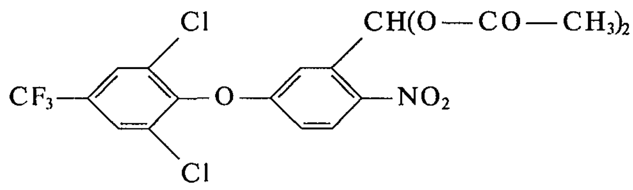

(IV-1)

34 g 3-(2,6-Dichlor-4-trifluormethyl-phenoxy)-benzaldehyd und 42,8 g Acetanhydrid werden in 100 ml Methylenchlorid gelöst, und die Lösung wird 1 Stunde bei 40°C gerührt. Nach dem Abkühlen auf 20°C werden 2 g Schwefelsäure dazugegeben. Dann werden bei 2—5°C 10 g 70%ige Salpetersäure zugetropft, und die Reaktionsmischung wird 4 Stunden bei 0—5°C gerührt. Zur Aufarbeitung wird mit 1 l Wasser verdünnt, das Methylenchlorid abdestilliert und vom kristallinen Produkt abgesaugt. Man erhält 80 g 5-(2,6-Dichlor-4-trifluormethyl-phenoxy)-2-nitro-benzaldehyd-diacetacylal vom Schmelzpunkt 132°C.

Beispiel V-1

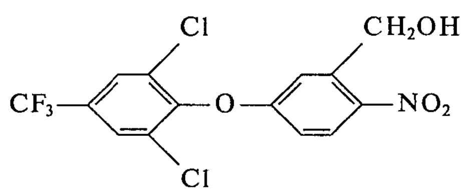

(V-1)

Eine Lösung von 41,5 g 5-(2,6-Dichlor-4-trifluormethyl-phenoxy)-2-nitro-benzoesäurechlorid in 100 ml Diglyme wird zu einem Gemisch aus 4,2 g Natriumboranat und 60 ml Diglyme bei −5 bis 10°C gegeben. Das Reaktionsgemisch wird ca. 15 Stunden bei Raumtemperatur gerührt, mit 1,2 l Wasser verdünnt, mit Essigsäure angesäuert und abgesaugt. Man erhält 36 g (94% der Theorie) 5-(2,6-Dichlor-4-trifluormethyl-phenoxy)-2-nitro-benzylalkohol vom Schmelzpunkt 143°C.

Beispiel VI-1

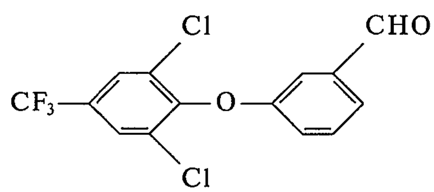

(VI-1)

Eine Mischung aus 123 g Kaliumhydroxid, 122 g 3-Hydroxybenzaldehyd und 1 l Methanol wird bis zur Bildung einer Lösung gerührt. Dann wird das Methanol im Vakuum sorgfältig abdestilliert. Der Rückstand wird in 1,8 l Dimethylsulfoxid gelöst. Dazu werden 300 ml Toluol gegeben und langsam wieder abdestilliert. Dabei werden 536 g 3,4,5-Trichlor-benzotrifluorid zugetropft. Das Reaktionsgemisch wird noch 9 Stunden bei 90—95°C gerührt. Anschließend wird das Lösungsmittel im Vakuum abdestilliert, der Rückstand mit 1,2 l Toluol verrührt und abgesaugt. Das Filtrat wird alkalisch und neutral gewaschen und eingeengt. Man erhält 430 g (64% der Theorie) 3-(2,6-Dichlor-4-trifluor-methylphenoxy)-benzaldehyd vom Schmelzpunkt 54°C.

Beispiel VII-1

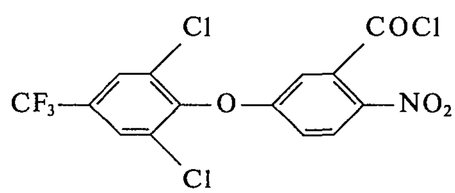

(VI-1)

79,2 g 5-(2,6-Dichlor-4-trifluormethyl-phenoxy)-2-nitrobenzoesäure und 0,5 ml Dimethylformamid werden in 150 ml 1,2-Dichlorethan vorgelegt. Dazu werden 28,6 g Thionylchlorid bei 60—65°C

tropfenweise gegeben, und die Mischung wird 1 Stunde unter Rückfluß erhitzt. Die Lösung wird mit Aktivkohle geklärt und über Kieselgur abgesaugt. Die Mutterlauge wird eingeengt und der Rückstand aus Cyclohexan umkristallisiert. Man erhält 65,1 g (79% der Theorie) 5-(2,6-Dichlor-4-trifluormethyl-phenoxy)-2-nitro-benzoesäurechlorid vom Schmelzpunkt 95°C.

## Anwendungsbeispiele

In diesen Beispielen werden die nachstehend angegebenen Stoffe eingesetzt:

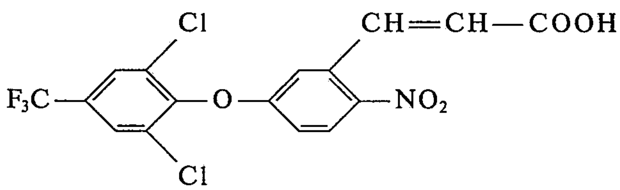

(I-1)

### Beispiel A

Pre-emergence-Test

Lösungsmittel: 5 Gewichtsteile Aceton
Emulgator: 1 Gewichtsteil Alkylarylpolyglycolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitunge spielt keine Rolle, entscheidend ist nur die Aufwandmenge des Wirkstoffs pro Flächeneinheit. Nach 3 Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Der erfindungsgemäße Wirkstoff (I-1) zeigt in diesem Test eine sehr gute selektive herbizide Wirksamkeit.

### Beispiel B

Post-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5—15 cm haben so, daß die jeweils gewünschte Wirkstoffmenge pro Flächeneinheit ausgebracht wird. Die Konzentration der Spritzbrühe wird so gewählt, daß in 2000 l Wasser/ha die jeweils gewünschte Wirkstoffmenge ausgebracht wird. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Der erfindungsgemäße Wirkstoff (I-1) zeigt in diesem Test eine sehr gute herbizide Wirksamkeit.

**Patentansprüche**

1. Substituierte Phenoxyzimtsäure-Derivate der Formel

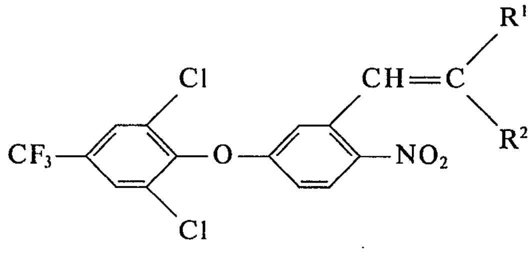

(I)

in welcher

$R^1$ für Wasserstoff, Cyano, $C_1$—$C_4$-Alkyl, Acetyl oder $C_1$—$C_4$-Alkoxycarbonyl steht und
$R^2$ für Cyano, $C_1$—$C_4$-Alkoxy-carbonyl oder COOM steht, wobei
M für Wasserstoff, Natrium, Kalium, ein Magnesium- oder Calciumionenäquivalent steht.

2. Verfahren zur Herstellung von substituierten Phenoxyzimtsäure-Derivaten der Formel

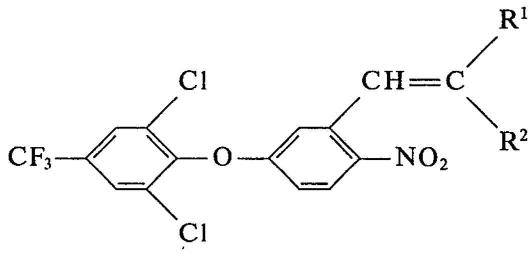

(I)

in welcher

$R^1$ für Wasserstoff, Cyano, $C_1$—$C_4$-Alkyl, Acetyl oder $C_1$—$C_4$-Alkoxycarbonyl steht und
$R^2$ für Cyano, $C_1$—$C_4$-Alkoxy-carbonyl oder COOM steht, wobei
M für Wasserstoff, Natrium, Kalium, ein Magnesium- oder Calciumionenäquivalent steht,

dadurch gekennzeichnet, daß man den substituierten Phenoxybenzaldehyd der Formel

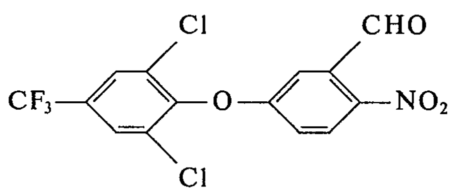

(II)

mit Methylenverbindungen der Formel

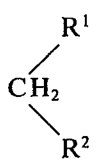

(III)

in welcher

$R^1$ und $R^2$ die oben angegebene Bedeutung haben und
$R^1$ außerdem auch für Carboxyl steht,

gegebenenfalls in Anwesenheit eines Katalysators und gegebenenfalls in Anwesenheit eines Verdünnungsmittels umsetzt und gegebenenfalls die hierbei gebildeten Verbindungen der Formel (I) nach üblichen Methoden zur Abwandlung der Reste $R^1$ und/oder $R^2$ in andere Verbindungen der Formel (I) umwandelt.

3. Herbizide Mittel, gekennzeichnet durch einen Gehalt an mindestens einem substituierten Phenoxyzimtsäurederivat der Formel (I).

4. Verwendung von substituierten Phenoxyzimtsäure-Derivaten der Formel (I) zur Bekämpfung von Unkräutern.

## Claims

1. Substituted phenoxycinnamic acid derivatives of the formula

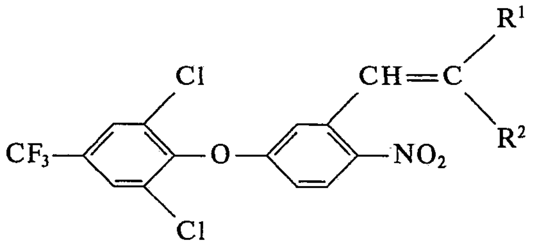

(I)

in which

$R^1$ represents hydrogen, cyano, $C_1$—$C_4$-alkyl, acetyl or $C_1$—$C_4$-alkoxycarbonyl and
$R^2$ represents cyano, $C_1$—$C_4$-alkoxycarbonyl or COOM, wherein
M represents hydrogen, sodium, potassium, or one equivalent of a magnesium or calcium ion.

2. Process for the preparation of substituted phenoxycinnamic acid derivates of the formula

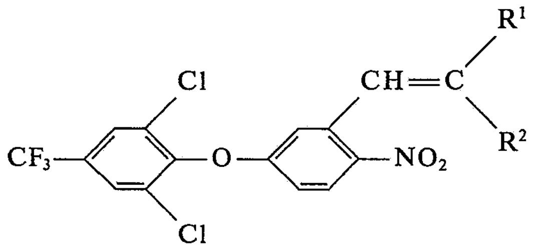

(I)

in which

$R^1$ represents hydrogen, cyano, $C_1$—$C_4$-alkyl, acetyl or $C_1$—$C_4$-alkoxycarbonyl and
$R^2$ represents cyano, $C_1$—$C_4$-alkoxycarbonyl or COOM, wherein
M represents hydrogen, sodium, potassium, or one equivalent of a magnesium or calcium ion,

characetised in that the substituted phenoxybenzaldehyde of the formula

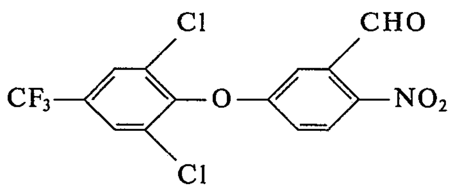

(II)

is reacted with methylene compounds of the formula

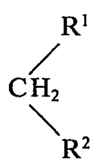

(III)

in which

$R^1$ and $R^2$ have the meaning given above and
$R^1$ also represents carboxyl,

if appropriate in the presence of a catalyst and if appropriate in the presence of a diluent and, if desired, the compounds of the formula (I) thus formed are converted into other compounds of the formula (I) by customary methods for modifying radicals $R^1$ and/or $R^2$.

3. Herbicidal agents, characterised in that they contain at least one substituted phenoxycinnamic acid derivative of the formula (I).

4. Use of substituted phenoxycinnamiac acid derivatives of the formula (I) for combating weeds.

**Revendications**

1. Dérivés substitués d'acide phénoxycinnamique de formule

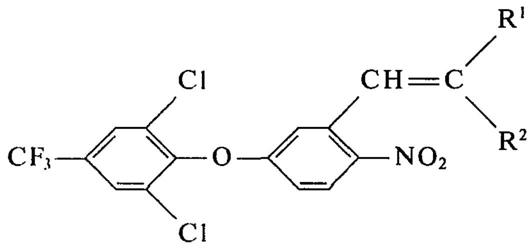

(I)

dans laquelle

$R^1$ représente l'hydrogène, un groupe cyano, alkyle en $C_1$—$C_4$, acétyle ou (alcoxy en $C_1$—$C_4$)-carbonyle et
$R^2$ est un groupe cyano, (alcoxy en $C_1$—$C_4$)-carbonyle ou COOM,
M représentant l'hydrogène, le sodium, le potassium, un équivalent d'ion magnésium ou un équivalent d'ion calcium.

2. Procédé de production de dérivés substitués d'acide phénoxycinnamique de formule

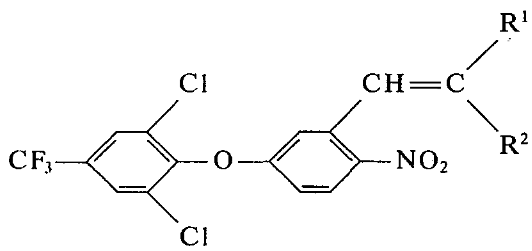

(I)

dans laquelle

$R^1$ représente l'hydrogène, un groupe cyano, alkyle en $C_1$—$C_4$, acétyle ou (alcoxy en $C_1$—$C_4$)-carbonyle et
$R^2$ est un groupe cyano, (alcoxy en $C_1$—$C_4$)-carbonyle ou COOM,
M représentant l'hydrogène, le sodium, le potassium, un équivalent d'ion magnésium ou un équivalent d'ion calcium,

caractérisé en ce qu'on fait réagir le phénoxybenzaldéhyde substitué de formule

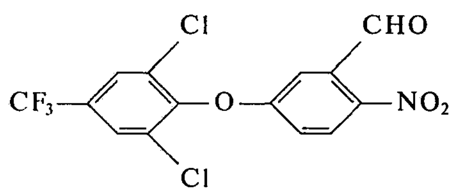

(II)

avec des composés méthyléniques de formule

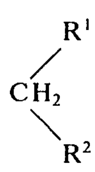

(III)

dans laquelle

$R^1$ et $R^2$ ont la définition indiquée ci-dessus et
$R^1$ représente en outre,

également, le groupe carboxyle, éventuellement en présence d'un catalyseur et le cas échéant en présence d'un diluant et on transforme éventuellement les composés ainsi formés de formule (I) par le procédé classique de modification des groupes $R^1$ et/ou $R^2$ en d'autres composés de formule (I).

3. Composition herbicide, caractérisée par une teneur en au moins un dérivé substitué d'acide phénoxycinnamique de formule (I).

4. Utilisation de dérivés substitués d'acide phénoxycinnamique de formule (I) pour combattre des mauvaises herbes.